## Europäisches Patentamt

## European Patent Office

## Office européen des brevets

(19)

(11) Veröffentlichungsnummer: **0 014 756 B1**

(12) **EUROPÄISCHE PATENTSCHRIFT**

(45) Veröffentlichungstag der Patentschrift:
14.07.82

(51) Int. Cl.³: **C 07 G  7/00,** A 61 K  37/02, G 01 N  33/48

(21) Anmeldenummer: **79105139.4**

(22) Anmeldetag: **13.12.79**

(54) Plazentaspezifisches Gewebsprotein PP10 und Verfahren zu seiner Anreicherung.

(30) Priorität: **19.12.78  DE 2854759**

(43) Veröffentlichungstag der Anmeldung:
**03.09.80 Patentblatt 80/18**

(45) Bekanntmachung des Hinweises auf die Patenterteilung:
**14.07.82 Patentblatt 82/28**

(84) Benannte Vertragsstaaten:
**AT BE CH DE FR GB IT LU NL SE**

(56) Entgegenhaltungen:
**keine**
**DE-A-2 640 387**
**DE-A-2 720 704**

(73) Patentinhaber: **BEHRINGWERKE AKTIENGESELLSCHAFT, Postfach 1140, D-3550 Marburg/Lahn (DE)**

(72) Erfinder: **Bohn, Hans, Dr., Oberer Eichweg 26, D-3550 Marburg (DE)**
Erfinder: **Kraus, Walter, Auf der Heide 15, D-3551 Bürgeln (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

## Plazentaspezifisches Gewebsprotein PP$_{10}$ und Verfahren zu seiner Anreicherung

Die Erfindung betrifft ein neues, plazentaspezifisches Protein (PP$_{10}$) und ein Verfahren zu dessen Anreicherung, insbesondere aus Plazentenextrakt.

In der deutschen Offenlegungsschrift 2 640 387 ist ein gewebespezifisches Protein beschrieben, das auch in Plazenta enthalten ist, jedoch mit dem hier beschriebenen nicht identisch ist.

Gegenstand der Erfindung ist das plazentaspezifische Protein PP$_{10}$, gekennzeichnet durch

a) einen Proteinanteil von 93±3%, einen Gehalt an Kohlenhydraten von 6,65±1,55% und davon Hexosen 4,8±1,0%, Hexosamin 1,2±0,3%, Fucose 0,05±0,05%, Neuraminsäure 0,6±0,2%;

b) einen Sedimentationskoeffizienten $S^{\circ}_{20,w}$ von 3,8±0,2 S;

c) ein in der Ultrazentrifuge bestimmtes Molekulargewicht von 48 000±5000;

d) ein im Natriumdodecylsulfat (SDS)-haltigen Polyacrylamidgel bestimmtes Molekulargewicht von 65 000±5000;

e) einen Extinktionskoeffizienten $E^{1\%}_{1cm}$ (280 nm) von 10,9±0,5;

f) eine elektrophoretische Beweglichkeit im Bereich der alpha$_1$-Globuline;

g) einen isoelektrischen Punkt von 5,1±0,3;

h) eine Aminosäurenzusammensetzung:

| | Mol-% | Variations-Koeffizient (%) |
|---|---|---|
| Lysin | 6,51 | 1,55 |
| Histidin | 1,81 | 5,21 |
| Arginin | 3,67 | 2,24 |
| Asparaginsäure | 9,82 | 0,79 |
| Threonin | 5,20 | 3,32 |
| Serin | 7,32 | 3,91 |
| Glutaminsäure | 11,50 | 0,54 |
| Prolin | 5,16 | 2,76 |
| Glycin | 6,97 | 1,71 |
| Alanin | 7,74 | 4,70 |
| Cystin/2 | 1,94 | 3,45 |
| Valin | 6,32 | 6,54 |
| Methionin | 2,43 | 4,54 |
| Isoleucin | 4,09 | 1,97 |
| Leucin | 9,46 | 2,63 |
| Tyrosin | 2,96 | 8,88 |
| Phenylalanin | 5,44 | 1,13 |
| Tryptophan | 1,69 | 7,04 |

und

i) die Fähigkeit, die Bildung spezifischer Antikörper auszulösen.

Zur Erläuterung der kennzeichnenden Merkmale des Gewebsproteins sei folgendes ausgeführt:

Die Bestimmung der Sedimentationskoeffizienten wurde in einer analytischen Ultrazentrifuge der Firma Beckmann (Spinco-Apparatur, Modell E) bei 60 000 UpM in Doppelsektorzellen mit Hilfe der UV-Scanner Technik bei 280 nm durchgeführt. Als Lösungsmittel diente ein 0,05 M Phospahtpuffer (pH 6,8), der 0,2 Mol/l NaCl enthielt. Die Proteinkonzentration wurde auf ca. 3 O.D.*) eingestellt. Die Sedimentationskoeffizienten sind auf die Basis von Wasser bei 20° C umgerechnet worden.

Zur Ermittlung des Molekulargewichts in der UZ wurde die Sedimentationsgleichgewichtsmethode herangezogen. Die Konzentration des Proteins ist dabei auf ca. 1.0 O.D. eingestellt worden. Die Bestimmung wurde bei 9000 UpM vorgenommen. Die Registrierung erfolgte mit UV-Optik bei 280 nm unter Einsatz des photoelektrischen Scanners.

Zur Bestimmung des Molekulargewichts im SDS-PAA-Gel wurde ein Gel mit 7,5% Polyacrylamid (PAA), das 0,1% Natriumdodecylsulfat (SDS) enthielt, verwendet. Als Vergleichssubstanz dienten humanes Plazentalaktogen (HPL) und Human-Albumin sowie dessen Aggregate.

Zur Bestimmung des Extinktionskoeffizienten wurde die Substanz 0,10%ig in Aqua dest. gelöst.

Die Untersuchung der elektrophoretischen Beweglichkeit erfolgte in der Mikromodifikation mit dem Gerät Microzone R 200 von Beckman Instruments auf Zelluloseazetatfolien (Fa. Sartorius) unter Verwendung von Natriumdiäthylbarbiturat-Puffer, pH 8,6.

Die Bestimmung des isoelektrischen Punktes wurde mit einer Säule (440 ml) der Firma LKB, Stockholm, durchgeführt. Das sogenannte Am- *) O.D. = optische Dichte.

pholin-Gemisch hatte bei der Untersuchung des Glykoproteins einen pH-Bereich von 4,0 bis 6,0.

Die Bestimmung der Kohlehydrate erfolgte nach der von H. E. Schultze, R. Schmidtberger, H. Haupt, Biochem. Z., 329, Seite 490 (1958), beschriebenen Methode.

Die Aminosäurenanalyse wurde nach S. Moore, D. H. Spackmann, W. H. Stein, Anal. Chem. 30, S. 1185 (1958), unter Verwendung des Flüssigkeitschromatographen Multichrom B der Firma Beckmann durchgeführt. 1/2-Cystin wurde nach Oxydation der Proteine mit Perameisensäure (S. Moore et al., Anal. Chem. 30, S. 1185 [1958]) und nachfolgender Chromatographie (S. Moore, J. Biol. Chem., 238, S. 235 [1963]) als Cysteinsäure bestimmt. Der Tryptophangehalt ist mit der direkten photometrischen Bestimmung nach H. Edelhoch, Biochemistry, 6, S. 1948 (1967), ermittelt worden.

Für $PP_{10}$ wurden folgende Eigenschaften festgestellt, die sich zur Isolierung des neuen Gewebsproteins verwenden lassen:

1) Mit Amoniumsulfat wird $PP_{10}$ bei pH 7,0 zwischen 30−60% Sättigung aus wäßrigen Lösungen gefällt.

2) Mit wasserlöslichen Akridinbasen, z. B. 2-Äthoxi-6,9-diaminoacridinlactat (Rivanol®) wird $PP_{10}$ bei pH-Werten zwischen 7−9 und einer Konzentration von 0,4 bis 0,8% w/v präzipitiert, nicht oder kaum dagegen bei pH 6,0, wenn die Rivanolkonzentration 0,4% beträgt.

3) Bei Fällungen mit Äthanol bleibt $PP_{10}$ in physiologischen Salzlösungen bei pH 7,0 bis zu einer Konzentration von 25% Alkohol im Überstand.

4) Bei der präparativen Elektrophorese wandert $PP_{10}$ im Bereich der $alpha_1$-Globuline.

5) Bei der Gelfiltration (Sephadex®) erscheint $PP_{10}$ im Bereich der Proteine mit Molekulargewichten von 30 000 bis 90 000.

6) $PP_{10}$ läßt sich an schwach basische Ionenaustauscher wie z. B. DEAE-Zellulose oder DEAE-Sephadex bei niedriger Leitfähigkeit (etwa 0−2 mS) und neutralem oder schwach alkalischem pH-Wert (etwa pH 7 bis 9) adsorbieren.

7) $PP_{10}$ kann aus seiner wäßrigen Lösung durch Immunadsorption angereichert und isoliert werden.

Gegenstand der Erfindung sind ferner Verfahren zur Herstellung des $PP_{10}$, dadurch gekennzeichnet, daß ein Extrakt aus der Plazenta, aber auch andere Lösungen, die dieses Protein enthalten, unter Zugrundelegung der obenstehenden Eigenschaften fraktioniert werden. Neben Ammoniumsulfat ist selbstverständlich, daß auch andere in der Präparativen Biochemie üblicherweise eingesetzte Neutralsalze zur Ausfällung des $PP_{10}$ verwendet werden können. Neben Akridinbasen sind auch wasserlösliche Derivate einer Chinolinbase, die bei Proteinfraktionierungen Verwendung finden, im Rahmen des erfindungsgemäßen Verfahrens einsetzbar. Entsprechend seinem elektrophoretischen Verhalten wie dem erfindungsgemäß festgestellten Molekulargewicht können zur Isolierung des Proteins auch andere Maßnahmen eingesetzt werden, die geeignet sind, ein $alpha_1$-Globulin von übrigen Plasma- oder Gewebeproteinen abzutrennen. Im Hinblick auf das Molekulargewicht könnten hierzu die verschiedenen Methoden der Gelfiltration, Gelchromatographie oder Ultrafiltration eingesetzt werden. Dies zeigt sich auch in der Eigenschaft des $PP_{10}$, an schwach basische Ionenaustauscher gebunden und hiervon wieder eluiert werden zu können.

Durch eine ausgewählte Kombination der genannten Maßnahmen, die einerseits zur Anreicherung des $PP_{10}$, andererseits zur Abtrennung dieses Proteins von den übrigen Gewebeproteinen bzw. Plasmaproteinen führen, kann die Isolierung der erfindungsgemäß gefundenen Substanz erfolgen. Demzufolge ist der Gegenstand der vorliegenden Erfindung in den einzelnen Anreicherungsschritten für das $PP_{10}$ und in den durch Kombination der Maßnahmen zur Anreicherung sich ergebenden Verfahren zur Reinigung des $PP_{10}$ zu sehen.

Das Verfahren zur Anreicherung ist gekennzeichnet durch die Anwendung mindestens einer der Maßnahmen 1 bis 7.

$PP_{10}$ hat antigene Eigenschaften; bei der Immunisierung von Tieren mit diesem Protein werden spezifische Antikörper gebildet. Der Nachweis und die Bestimmung von $PP_{10}$ mit immunologischen Methoden hat diagnostische Bedeutung: einerseits zur Überwachung von Schwangerschaften, andererseits zum Nachweis, speziell von trophoblastischen, aber auch von nicht-trophoblastischen Tumoren sowie zur Überwachung des Krankheitsverlaufs und zur Kontrolle der Therapie bei solchen Erkrankungen.

Die Erfindung wird am nachstehenden Beispiel näher erläutert:

### Beispiel

### A) Extraktion der Plazenten und Fraktionierung des Extraktes mit Rivanol und Ammoniumsulfat

1000 kg tiefgefrorene menschliche Plazenten werden im Schneidmischer zerkleinert und mit 1000 l einer 0,4%igen Kochsalzlösung extrahiert. Der Extrakt wird dann, nach Abtrennung des Geweberückstandes durch Zentrifugation, mit 20%iger Essigsäure auf pH 6,0 eingestellt und unter Rühren mit 200 l einer 3%igen Lösung von 2-Äthoxy-6,9-Diaminoacridin-Lactat (Rivanol®, Hoechst AG) versetzt. Der entstehende Niederschlag wird abzentrifugiert und verworfen. Zum Überstand gibt man 1% w/v Bentonit A (Fa. Erbslöh & Co., Geisenheim/Rhein), stellt den pH-Wert durch Zugabe von 2 H NaOH auf 7,0 ein und filtriert. Das Filtrat wird unter Rühren langsam mit 30% w/v Ammoniumsulfat versetzt; das Plazentaprotein PP$_{10}$ fällt dabei, zusammen mit anderen Proteinen, aus. Man filtriert den Niederschlag ab und erhält ca. 12 kg einer feuchten Paste, die im nachfolgenden als Fraktion A bezeichnet wird. 500 g dieser Paste enthalten im Durchschnitt etwa 250 mg PP$_{10}$.

## B) Gelfiltration an Sephadex G-150

500 g der Fraktion A werden in ca. 400 ml Wasser gelöst und gegen einen 0,1 M Tris-HCl-Puffer (pH 8,0), der 1,0 Mol/l NaCl und 0,1% NaN$_3$ enthält (Pufferlösung I) dialysiert. Die proteinhaltige Lösung wird auf eine mit Sephadex G-150 gefüllte Säule (20 × 100 cm) aufgetragen und gelfiltriert. Zum Eluiren wird die Pufferlösung I verwendet. Die Eluate testet man im Geldiffusionstest nach Ouchterlony mit einem spezifischen Anti-PP$_{10}$ Kaninchenserum; alle das plazentaspezifische Protein PP$_{10}$ enthaltenden Fraktionen werden gesammelt und auf einem Ultrafilter (Amicon UF 2000) unter Verwendung von PM 10-Membranen auf ca. 3000 ml eingeengt. Diese Lösung (Fraktion B) enthält insgesamt etwa 100 mg PP$_{10}$.

## C) Anreicherung von PP$_{10}$ durch Immunadsorption

### 1. Herstellung des Immunadsorbens

470 ml eines Anti-PP$_{10}$-Serums vom Kaninchen werden gegen 0,02 M Phosphatpuffer (pH 7,0) dialysiert und zur Abtrennung der Immunglobuline an DEAE-Zellulose chromatographiert. Die Immunglobulinfraktion (3,23 g Protein) wird dann mit 323 g besonders gereinigter Agarose in Kugelform (Sepharose® 4 B der Pharmacia, Uppsala, Schweden), die mit 40,5 g Bromcyan aktiviert worden war, umgesetzt und so kovalent an einem Träger gebunden.

Das Verfahren ist beschrieben von Axen, R., Porath, J., Ernbach A., Nature, 214, S. 1302 (1967).

Mit Hilfe eines auf diese Weise hergestellten Immunadsorbens kann das Plazentaprotein PP$_{10}$ aus dessen Lösung, insbesondere aus PP$_{10}$-angereicherten Plazentaextraktfraktionen isoliert werden.

### 2. Durchführung der Immunadsorption

Das Immunadsorbens wird in Pufferlösung I (0,1 M Tris-HCl-Puffer, pH 8,0, mit 1,0 Mol/l NaCl und 0,1% NaN$_3$) suspendiert, dann in eine Chromatographiesäule gefüllt (5,5 × 20 cm) und mit Pufferlösung I nachgespült. Dann läßt man langsam 60 ml der PP$_{10}$-haltigen Lösung (Fraktion B) durch die Säule wandern, wobei PP$_{10}$ immunadsorptiv gebunden wird. Man wäscht die Säule gründlich mit Puffer I nach und eluiert dann das adsorbierte Protein mit ca. 600 ml 3 M Kaliumrhodanid-Lösung. Die PP$_{10}$-haltigen Eluate werden gegen Pufferlösung I dialysiert und im Ultrafilter auf ca. 15 ml eingeengt. Ausbeute pro Adsorption 6 − 7 mg PP$_{10}$.

Das Adsorbens in der Säule wird unmittelbar nach der Elution von PP$_{10}$ wieder mit Pufferlösung I neutralisiert und gründlich gewaschen; es kann dann erneut zur immunadsorptiven Bindung von PP$_{10}$ eingesetzt werden.

## D) Hochreinigung von PP$_{10}$

Das durch Immunadsorption gewonnene Protein ist häufig durch unspezifisch gebundene Serumproteine und andere Plazenta-Gewebsproteine verunreinigt. Die Abtrennung der Hauptmenge der Serum-Begleitproteine gelingt z. B. durch Gelfiltration an Sephadex G-150 oder durch Chromatographie an DEAE-Zellulose. Die restlichen Begleitproteine werden dann durch inverse oder negative Immunadsorptions, d. h. mit Hilfe von trägergebundenen Antikörpern gegen die noch als Verunreinigung vorliegenden Proteine, entfernt.

**Patentansprüche für die Vertragsstaaten BE, CH, DE, FR, GB, IT, LU, NL, SE**

1. Gewebsprotein PP$_{10}$, gekennzeichnet durch

    a) einen Proteinanteil von 93±3%, einen Gehalt an Kohlenhydraten von 6,65±1,55% und davon Hexosen 4,8±1,0%, Hexosamin 1,2±0,3%, Fucose 0,05±0,05%, Neuraminsäure 0,6±0,2%;

    b) einen Sedimentationskoeffizienten $S^0_{20,w}$ von 3,8±0,2 S;

    c) ein in der Ultrazentrifuge bestimmtes Molekulargewicht von 48 000±5000;

    d) ein im Natriumdodecylsulfat (SDS)-haltigen Polyacrylamidgel bestimmtes Molekulargewicht von 65 000±5000;

    e) einen Extinktionskoeffizienten $E^{1\%}_{1cm}$ (280 nm) von 10,9±0,5;

    f) eine elektrophoretische Beweglichkeit im Bereich der alpha$_1$-Globuline;

    g) einen isoelektrischen Punkt von 5,1±0,3;

    h) eine Aminosäurenzusammensetzung:

|  | Mol-% | Variations-Koeffizient (%) |
|---|---|---|
| Lysin | 6,51 | 1,55 |
| Histidin | 1,81 | 5,21 |
| Arginin | 3,67 | 2,24 |
| Asparaginsäure | 9,82 | 0,79 |

| | Mol-% | Variations-Koeffizient (%) |
|---|---|---|
| Threonin | 5,20 | 3,32 |
| Serin | 7,32 | 3,91 |
| Glutaminsäure | 11,50 | 0,54 |
| Prolin | 5,16 | 2,76 |
| Glycin | 6,97 | 1,71 |
| Alanin | 7,74 | 4,70 |
| Cystin/2 | 1,94 | 3,45 |
| Valin | 6,32 | 6,54 |
| Methionin | 2,43 | 4,54 |
| Isoleucin | 4,09 | 1,97 |
| Leucin | 9,46 | 2,63 |
| Tyrosin | 2,96 | 8,88 |
| Phenylalanin | 5,44 | 1,13 |
| Tryptophan | 1,69 | 7,04 |

und
i) die Fähigkeit, die Bildung spezifischer Antikörper auszulösen.

2. Verfahren zur Anreicherung des Gewebsproteins PP$_{10}$ nach Anspruch 1, dadurch gekennzeichnet, daß Plazentenextrakt oder Lösungen, die dieses Protein enthalten, mindestens einer der folgenden Maßnahmen unterworfen werden und die bezüglich des Gewebsproteins PP$_{10}$ angereicherte Fraktion gewonnen wird:

1. Fällung mit Ammoniumsulfat im pH-Bereich von 5 – 8 zwischen 30 – 60% Sättigung;
2. Ausfällung von Begleitproteinen mit einer wasserlöslichen Akridinbase bei einem schwach sauren pH-Wert und bei einer Konzentration = 0,4% w/v, wobei PP$_{10}$ im Überstand bleibt;
3. Ausfällung von Begleitproteinen mit Äthanol bis zu einer Konzentration von 25% bei pH 7,0, wobei PP$_{10}$ im Überstand bleibt;
4. präparative Zonenelektrophorese und Gewinnung der alpha$_1$-Globulinfraktion;
5. Gelfiltration zur Gewinnung von Proteinen im Molekulargewichtsbereich von 30 000 bis 90 000;
6. Adsorption an schwach basische Ionenaustauscher und Elution des Gewebsproteins;
7. immunadsorptive Anreicherung.

**Patentanspruch für den Vertragsstaat AT**

Verfahren zur Anreicherung des Gewebsproteins PP$_{10}$, das charakterisiert ist durch

a) einen Proteinanteil von 93 ± 3%, einen Gehalt an Kohlenhydraten von 6,65 ± 1,55% und davon Hexosen 4,8 ± 1,0%, Hexosamin 1,2 ± 0,3%, Fucose 0,05 ± 0,05%, Neuraminsäure 0,6 ± 0,2%,
b) einen Sedimentationskoeffizienten $S^o_{20,w}$ von 3,8 ± 0,2 S,
c) ein in der Ultrazentrifuge bestimmtes Molekulargewicht von 48 000 ± 5000,
d) ein im Natriumdodecylsulfat (SDS)-haltigen Polyacrylamidgel bestimmtes Molekulargewicht von 65 000 ± 5000,
e) einen Extinktionskoeffizienten $E^{1\%}_{1cm}$ (280 nm) von 10,9 ± 0,5,
f) eine elektrophoretische Beweglichkeit im Bereich der alpha$_1$-Globuline,
g) einen isoelektrischen Punkt von 5,1 ± 0,3,
h) eine Aminosäurenzusammensetzung:

| | Mol-% | Variations-Koeffizient (%) |
|---|---|---|
| Lysin | 6,51 | 1,55 |
| Histidin | 1,81 | 5,21 |
| Arginin | 3,67 | 2,24 |
| Asparaginsäure | 9,82 | 0,79 |
| Threonin | 5,20 | 3,32 |
| Serin | 7,32 | 3,91 |
| Glutaminsäure | 11,50 | 0,54 |
| Prolin | 5,16 | 2,76 |
| Glycin | 6,97 | 1,71 |
| Alanin | 7,74 | 4,70 |
| Cystin/2 | 1,94 | 3,45 |
| Valin | 6,32 | 6,54 |
| Methionin | 2,43 | 4,54 |
| Isoleucin | 4,09 | 1,97 |
| Leucin | 9,46 | 2,63 |
| Tyrosin | 2,96 | 8,88 |
| Phenylalanin | 5,44 | 1,13 |
| Tryptophan | 1,69 | 7,04 |

und

i) die Fähigkeit, die Bildung spezifischer Antikörper auszulösen,

dadurch gekennzeichnet, daß man einen Plazentenextrakt oder Lösungen, die dieses Protein enthalten, bei einem pH zwischen 5 und 8 bis zu einer Sättigung zwischen 30 und 60% mit Ammoniumsulfat versetzt und den Niederschlag gewinnt und/oder mit 0,4% (w : v) einer wasserlöslichen Akridinbase in schwach saurer Lösung versetzt und den Überstand gewinnt und/oder mit Äthanol bis zu einer Konzentration von 25% bei pH 7,0 versetzt und den Überstand gewinnt und/oder einer präparativen Zonenelektrophorese unterwirft und die alpha$_1$-Globulinfraktion gewinnt und/oder einer Gelfiltration unterwirft und die Proteine mit Molekulargewichten zwischen 30 000 und 90 000 gewinnt und/oder an einen schwach basischen Ionenaustauscher adsorbiert und das adsorbierte Material eluiert und/oder einer Immunadsorption unterwirft.

**Claims for the Contracting States:**
**BE, CH, DE, FR, GB, IT, LU, NL, SE**

1. Tissue protein PP$_{10}$, characterized by

a) a protein proportion of $93\pm3\%$, a content of carbohydrates of $6.65\pm1.55\%$, and thereof hexoses $4.8\pm1.0\%$, hexosamine $1.2\pm0.3\%$, fucose $0.05\pm0.05\%$, sialic acid $0.6\pm0.2\%$;

b) a sedimentation coefficient $S^o_{20,w}$ of $3.8\pm0.2\,S$;

c) a molecular weight determined in the ultracentrifuge of $48\,000\pm5000$;

d) a molecular weight determined in sodium didecylsulfate (SDS)-containing polyacrylamide gel of $65\,000\pm5000$;

e) an extinction coefficient $E^{1\%}_{1\,cm}$ (280 nm) of $10.9\pm0.5$;

f) an electrophoretic mobility in the range of alpha$_1$-globulins;

g) an isoelectric point of $5.1\pm0.3$;

h) the amino acid composition

| | Mole % | Variation-coefficient (%) |
|---|---|---|
| Lysine | 6,51 | 1,55 |
| Histidine | 1,81 | 5,21 |
| Arginine | 3,67 | 2,24 |
| Aspartic acid | 9,82 | 0,79 |

| | Mole % | Variation-coefficient (%) |
|---|---|---|
| Threonine | 5,20 | 3,32 |
| Serine | 7,32 | 3,91 |
| Glutamic acid | 11,50 | 0,54 |
| Proline | 5,16 | 2,76 |
| Glycine | 6,97 | 1,71 |
| Alanine | 7,74 | 4,70 |
| Cystine/2 | 1,94 | 3,45 |
| Valine | 6,32 | 6,54 |
| Methionine | 2,43 | 4,54 |
| Isoleucine | 4,09 | 1,97 |
| Leucine | 9,46 | 2,63 |
| Tyrosine | 2,96 | 8,88 |
| Phenylalanine | 5,44 | 1,13 |
| Tryptophan | 1,69 | 7,04 |

and

i) the property to induce the formation of specific antibodies.

2. Process for enriching the tissue protein PP$_{10}$ claimed in claim 1, which comprises subjecting the extract of placentas or solutions which contain this protein to at least one of the hereinafter described measures and isolating the fraction enriched with the tissue protein PP$_{10}$:

1. precipitation with ammonium sulfate in a pH range of from 5 to 8 at a saturation of 30 to 60%;

2. precipitation of accompanying proteins with a water-soluble acridine base at a weakly acid pH-value and a concentration of 0.4% w/v, PP$_{10}$ remaining in the supernatant;

3. Precipitation of accompanying proteins with ethanol at a concentration of 25% at pH 7,0, PP$_{10}$ remaining in the supernatant;

4. Preparative zone electrophoresis and isolation of the alpha$_1$-globulin fraction;

5. Gel-filtration for the isolation of proteins having a molecular weight in the range of 30 000 to 90 000;

6. Adsorption on weakly basic ion exchangers and elution of the tissue protein;

7. Enrichment by immuno-adsorption.

**Claim for the Contracting State: AT**

Process for enriching the tissue protein PP$_{10}$ characterized by

a) a protein proportion of 93±3%, a content of carbohydrates of 6.65±1.55%, and thereof hexoses 4.8±1.0%, hexosamine 1.2±0.3%, fucose 0.05±0.05%, sialic acid 0.6±0.2%;

b) a sedimentation coefficient S$^{\circ}_{20,w}$ of 3.8±0.2 S;

c) a molecular weight determined in the ultracentrifuge of 48 000±5000;

d) a molecular weight determined in sodium dodecylsulfate (SDS)-containing polyacrylamide gel of 65 000±5000;

e) an extinction coefficient E$^{1\%}_{1cm}$ (280 nm) of 10.9±0.5;

f) an electrophoretic mobility in the range of alpha$_1$-globulins;

g) an isoelectric point of 5.1±0.3;

h) the amino acid composition

|  | Mole % | Variation-coefficient (%) |
|---|---|---|
| Lysine | 6,51 | 1,55 |
| Histidine | 1,81 | 5,21 |
| Arginine | 3,67 | 2,24 |
| Aspartic | 9,82 | 0,79 |
| Threonine | 5,20 | 3,32 |
| Serine | 7,32 | 3,91 |
| Glutamic acid | 11,50 | 0,54 |
| Proline | 5,16 | 2,76 |
| Glycine | 6,97 | 1,71 |
| Alanine | 7,74 | 4,70 |
| Cystine/2 | 1,94 | 3,45 |
| Valine | 6,32 | 6,54 |
| Methionine | 2,43 | 4,54 |
| Isoleucine | 4,09 | 1,97 |
| Leucine | 9,46 | 2,63 |
| Tyrosine | 2,96 | 8,88 |
| Phenylalanine | 5,44 | 1,13 |
| Tryptophan | 1,69 | 7,04 |

and

i) the property to induce the formation of specific antibodies,

which comprises adding to an extract from placenta or a solution containing that protein ammonium sulfate at a pH of between 5 and 8 up to a saturation of between 30 and 60% and separating the precipitate and/or adding 0.4% (w : v) of a water-suluble acridine base to the precipitate in a weakly acidic solution and separating the supernatant and/or adding ethanol up to a concentration of 25% at pH 7,0 and separating the supernatant and/or performing a preparative zone electrophoresis and separating the alpha$_1$-globulin fraction and/or performing a gel filtration and separating the proteins with molecular weights of between 30 000 and 90 000 and/or adsorption to a weakly basic ion exchanger and elution the adsorbed material and/or performing an immuno-adsorption.

**Revendications pour les Etats contractants: BE,CH, DE, FR, GB, IT, LU, NL, SE**

1. Protéine tissulaire PP$_{10}$, caractérisée par

a) une teneur en protéines de 93±3%, une teneur en hydrates de carbone de 6,65±1,55% dont hexoses 4,8±1,0%, hexosamine 1,2±0,3%, fucose 0,05±0,05%, acide neuraminique 0,6±0,2%;

b) un coefficient de sédimentation S$^{\circ}_{20,w}$ de 3,8±0,2 S;

c) une masse moléculaire, déterminée à l'ultracentrifugeuse, de 48 000±5000;

d) une masse moléculaire déterminée dans un gel de polyacrylamide contenant du dodécylsulfate de sodium (SDS) de 65 000±5000;

e) un coefficient d'extinction E$^{1\%}_{1cm}$ (280 nm) de 10,9±0,5;

f) une mobilité électrophorétique dans le domaine des alpha 1-globulines;

g) un point isoélectrique de 5,1±0,3;

h) une composition en amino-acides:

|  | moles % | coefficient de variation (%) |
|---|---|---|
| Lysine | 6,51 | 1,55 |
| Histidine | 1,81 | 5,21 |
| Arginine | 3,67 | 2,24 |
| Acide aspartique | 9,82 | 0,79 |
| Thréonine | 5,20 | 3,32 |

## Fortsetzung

| | moles % | coefficent de variation (%) |
|---|---|---|
| Sérine | 7,32 | 3,91 |
| Acide glutamique | 11,50 | 0,54 |
| Proline | 5,16 | 2,76 |
| Glycine | 6,97 | 1,71 |
| Alanine | 7,74 | 4,70 |
| Cystine/2 | 1,94 | 3,45 |
| Valine | 6,32 | 6,54 |
| Méthionine | 2,43 | 4,54 |
| Isoleucine | 4,09 | 1,97 |
| Leucine | 9,46 | 2,63 |
| Tyrosine | 2,96 | 8,88 |
| Phénylalanine | 5,44 | 1,13 |
| Tryptophane | 1,69 | 7,04 |

et

i) la capacité de déclencher la formation d'anticorps spécifiques.

2. Procédé d'enrichissement de la protéine tissulaire PP$_{10}$ suivant la revendication 1, caractérisé en ce que l'extrait placentaire ou les solutions qui contiennent cette protéine, sont soumis à au moins une des mesures suivantes et l'on récupère la fraction enrichie en protéine tissulaire PP$_{10}$:

1. Précipitation par le sulfate d'ammonium dans le domaine de pH de 5 à 8 entre 30 et 60% de la saturation;
2. Précipitation des protéines présentes comme impuretés avec une base acridinique soluble dans l'eau à un pH faiblement acide et à une concentration de 0,4% P/V, la PP$_{10}$ restant dans le liquide surnageant;
3. Précipitation des protéines présentes comme d'impuretés par l'éthanol jusqu'à une concentration de 25% à pH 7,0, la PP$_{10}$ restant dans le liquide surnageant;
4. Electrophorèse de zone préparative et récupération de la fraction alpha$_1$-globuline;
5. Filtration sur gel pour la récupération de protéines dans le domaine de masses moléculaires de 30 000 à 90 000;

6. Adsorption sur des échangeurs d'ions faiblement basiques et élution de la protéine tissulaire;
7. Enrichissement par immunoadsorption.

**Revendication pour l'etat contractant: AT**

Procédé d'enrichissement de la protéine tissulaire PP$_{10}$, caractérisé par

a) une teneur en protéines de $93\pm3\%$, une teneur en hydrates de carbone de $6,65\pm1,55\%$ dont hexoses $4,8\pm1,0\%$, hexos-amine $1,2\pm0,3\%$, fucose $0,05\pm0,05\%$, acide neuraminique $0,6\pm0,2\%$;
b) un coefficient de sédimentation $S^{\circ}_{20,w}$ de $3,8\pm0,2$ S;
c) une masse moléculaire, déterminée à l'ultra-centrifugeuse, de $48\,000\pm5000$;
d) une masse moléculaire déterminée dans un gel de polyacrylamide contenant du dodécylsulfate de sodium (SDS) de $65\,000\pm5000$;
e) un coefficient d'extinction $E^{1\%}_{1cm}$ (280 nm) de $10,9\pm0,5$;
f) une mobilité électrophorétique dans le domaine des alpha 1-globulines;
g) un point isoélectrique de $5,1\pm0,3$;
h) une composition en amino-acides:

| | moles % | coefficient de variation (%) |
|---|---|---|
| Lysine | 6,51 | 1,55 |
| Histidine | 1,81 | 5,21 |
| Arginine | 3,67 | 2,24 |
| Acide aspartique | 9,82 | 0,79 |
| Thréonine | 5,20 | 3,32 |
| Sérine | 7,32 | 3,91 |
| Acide glutamique | 11,50 | 0,54 |
| Proline | 5,16 | 2,76 |
| Glycine | 6,97 | 1,71 |
| Alanine | 7,74 | 4,70 |
| Cystine/2 | 1,94 | 3,45 |
| Valine | 6,32 | 6,54 |
| Méthionine | 2,43 | 4,54 |
| Isoleucine | 4,09 | 1,97 |
| Leucine | 9,46 | 2,63 |

Fortsetzung

|  | moles % | coefficient de variation (%) |
|---|---|---|
| Tyrosine | 2,96 | 8,88 |
| Phénylalanine | 5,44 | 1,13 |
| Tryptophane | 1,69 | 7,04 |

et

i) la capacité de déclencher la formation d'anticorps spécifiques,

procédé caractérisé en ce que l'on ajoute à un extrait placentaire ou aux solutions qui contiennent cette protéine, dans le domaine de pH de 5 à 8, du sulfate d'ammonium jusqu'à une saturation de 30 à 60% et on récupère le précipité et/ou on ajoute 0.4% (P : V) d'une base acridinique soluble dans l'eau en solution faiblement acide et on récupère le liquide surnageant et/ou on ajoute de l'éthanol jusqu'à une concentration de 25% à un pH de 7.0 et on récupère le liquide surnageant et/ou on soumet ledit extrait placentaire ou les solutions qui contiennent cette protéine à une électrophorèse de zone préparative et on récupère la fraction alpha$_1$-globuline et/ou on soumet à une filtration sur gel et on récupère les protéines dans le domaine de masses moléculaires de 30 000 à 90 000 et/ou on adsorbe sur un échangeur d'ions faiblement basique et on élue la matière adsorbée et/ou on soumet à une immunoadsorption.